# EUROPÄISCHE PATENTSCHRIFT

(11) **EP 3 598 946 B1**
(45) Veröffentlichungstag und Bekanntmachung des Hinweises auf die Patenterteilung: **16.10.2024**
(21) Anmeldenummer: 18185658.4
(22) Anmeldetag: 26.07.2018
(51) Int. Cl.: A61B 6/00, A61B 6/04, A61B 8/00, A61B 8/08, A61B 6/50

(54) **ERMITTLUNG EINES DARZUSTELLENDEN INTERESSIERENDEN BEREICHS**
IDENTIFICATION OF A REGION OF INTEREST TO BE REPRESENTED
DÉTERMINATION D'UNE ZONE INTÉRESSANTE À REPRÉSENTER

(43) Veröffentlichungstag der Anmeldung: 29.01.2020
(73) Patentinhaber: Siemens Healthineers AG, 91301 Forchheim (DE)
(72) Erfinder: Radicke, Marcus, 90587 Veitsbronn (DE); Ritschl, Ludwig, 91054 Erlangen (DE)
(74) Vertreter: Siemens Healthineers Patent Attorneys

(56) Entgegenhaltungen:
- EP-A1- 3 219 261
- EP-A1- 3 336 803
- BENEDIKT SCHAEFGEN ET AL: "Initial results of the FUSION-X-US prototype combining 3D automated breast ultrasound and digital breast tomosynthesis", EUROPEAN RADIOLOGY, vol. 28, no. 6, 4 January 2018 (2018-01-04), DE, pages 2499 - 2506, XP055537415, ISSN: 0938-7994, DOI: 10.1007/s00330-017-5235-8

## Beschreibung

Die Erfindung betrifft ein Verfahren zur Ermittlung eines darzustellenden interessierenden Bereichs, ein Verfahren zur Darstellung eines interessierenden Bereichs, eine Bildverarbeitungseinrichtung und ein Röntgensystem.

Medizinische Untersuchungen einer Brust, insbesondere die einer menschlichen weiblichen Brust, werden in der Regel durchgeführt, um bösartige Veränderungen des Brustgewebes gezielt und sicher feststellen bzw. diagnostizieren zu können. Eine gängige Methode ist die Mammographie, um solch eine Untersuchung durchführen zu können.

Bei einer mammographischen Untersuchung wird die Brust in einem Röntgengerät bzw. in einem Mammographiegerät zwischen zwei Kompressionsplatten positioniert und komprimiert und in diesem Zustand ein Röntgenbild erzeugt. Dazu werden mittels einer Röntgenquelle Röntgenstrahlen ausgesandt. Diese werden beim Passieren der Brust je nach Art und Struktur des durchdrungenen Gewebes in ihrer Intensität geschwächt und anschließend als Mammogramm von einem Detektor erfasst.

Je nach Befund oder Gewebebeschaffenheit der zu untersuchenden Brust kann es von Vorteil sein, noch zusätzliche bzw. ergänzende Informationen zu dem zu untersuchenden Brustgewebe zu ermittelten. In der Praxis wird daher häufig mittels Tomosynthese oder Ultraschall ein Volumenbild der Brust aufgenommen. Ein Nachteil der Tomosynthese ist dabei jedoch, dass der Patient einer erhöhten Strahlungsbelastung ausgesetzt wird.

Bei einer Ultraschallaufnahme bzw. Sonographieaufnahme werden mittels der Ultraschallsonde Ultraschallwellen in Richtung eines zu untersuchenden Objekts, wie beispielsweise der Brust einer Patientin, ausgesendet. Die Ultraschallwellen werden an den unterschiedlichen Strukturen des zu untersuchenden Objekts gestreut, gebrochen und reflektiert. Die zurückkommenden Ultraschallwellen werden von der Ultraschallsonde wieder empfangen und anschließend ausgewertet und umgewandelt, so dass ein Bild des Inneren des zu untersuchenden Objekts erzeugt werden kann. Diese bildliche Darstellung kann dabei sowohl 2-dimensional als auch 3-dimensional erfolgen.

Um die unterschiedlichen Techniken, Ultraschall und Röntgenmammographie, zweckdienlich zu verbinden und eine Befundung möglichst einfach zu gestalten, werden in der Praxis Kombinationsgeräte eingesetzt. Bei einem solchen kombinierten Röntgen-3D-Ultraschall-Gerät (im Folgenden kurz XUS-Gerät) ist eine Ultraschallsonde der Ultraschallanordnung in der Regel zwischen der Röntgenquelle und dem Detektor des Röntgengeräts angeordnet. Sie kann dabei grundsätzlich sowohl auf der zum Detektor weisenden Seite des zu untersuchenden Objekts als auch auf dessen zur Röntgenquelle weisenden Seite angeordnet sein. Die Ultraschallsonde, der Detektor und die Kompressionsplatten sind in der Regel parallel zueinander angeordnet. Mittels dieser Anordnung können also Aufnahmen mit im Wesentlichen ähnlichen Ansichten des Untersuchungsobjekts unter Verwendung der zwei unterschiedlichen Bildgebungsverfahren erzeugt werden.

Eine örtliche Zuordnung zwischen dem zweidimensionalen Röntgenbild (Mammogramm) und einer Position in einem dreidimensionalen Ultraschallbild bzw. Ultraschall-Volumendatensatz gestaltet sich dabei jedoch schwierig. Wenn zum Beispiel ein Befunder in dem Röntgenbild eine Position ermittelt hat, die in einem interessierenden Bereich liegt, ist eine eindeutige Zuordnung der Position zu einer entsprechenden Position im Ultraschallbild nicht möglich. Denn aufgrund der einem Projektionsbild innewohnenden Eigenschaft fehlen die räumlichen Informationen. Erschwerend kommt hinzu, dass der Röntgenstrahlengang eine Kegelform aufweist. Dadurch können einer Position im Röntgenprojektionsbild bzw. einem Pixel des Röntgendetektors nicht einfach die Elemente im dreidimensionalen Ultraschallbild zugeordnet werden, die den Strukturen des Untersuchungsobjekts entsprechen, die auf einer durch das Pixel verlaufenden Senkrechten zur Detektorfläche liegen.

Um eine genaue Positionsbestimmung vorzunehmen wird in der Praxis häufig ein zweites Röntgenprojektionsbild in einem definierten Winkel zum ersten Röntgenprojektionsbild aufgenommen. Damit lässt sich eine genaue Position in Bezug zu dem XUS-Gerät und somit auch in dem dreidimensionalen Ultraschallbild ermitteln. Dies ist jedoch aufgrund der erhöhten Strahlenbelastung für den Patienten nachteilig.

Grundsätzlich ist auch eine Transformation des Koordinatensystems des Ultraschall-Volumendatensatzes möglich. Dazu wird der Ultraschall-Volumendatensatz mithilfe von Standardmethoden der Transformation und Interpolation in die Kegelgeometrie des Röntgenstrahlengangs transformiert. Der Ultraschall-Volumendatensatz korreliert dann mit dem Röntgenprojektionsbild, sodass eine Visualisierung ohne weiteres möglich ist. Allerdings liegt der Ultraschall-Volumendatensatz nach der Transformation in einem Zerrbild vor, wodurch eine korrekte Befundung unmöglich oder zumindest stark erschwert wird.

Aus der Druckschrift EP 3 336 803 A1 ist ein Bildüberprüfungsverfahren und ein System zur Durchführung des Verfahrens bekannt, das die im Referenzbild oder in den synthetischen Ansichten und/oder den vom Arzt überprüften Slabs angezeigten Informationen mit farbigen Informationsschichten anreichert, die aus einem Bildvolumen gewonnen und mit dem Referenzbild, den Slabs und/oder den synthetischen Ansichten zusammengeführt werden. Die Anreicherung des Referenzbildes, der Slabs und/oder der synthetischen Ansichten mit farbigen Schichten hebt die farbigen Bereiche von Interesse oder Merkmale des Bildvolumens in den Slabs und/oder den synthetischen Ansichten auf eine Weise hervor, die verhindert, dass die Merkmale aufgrund von Gewebeüberlagerungseffekten oder anderen Problemen bei der Kombination der farbigen Schichten und des Referenzbildes zur Bildung der angezeigten kombinierten Bilder verloren gehen.

Aus der Veröffentlichung BENEDIKT SCHAEFGEN et.al.: "Initial results of the FUSION-X-US prototype combining 3D automated breast ultrasound and digital breast tomosynthesis ", EUROPE-AN RADIOLOGY, Bd. 28, Nr. 6, 4. Januar 2018 (2018-01-04), Seiten 2499-2506, XP055537415, DE ISSN: 0938-7994, DOI: 10.1007/s00330-017-5235-8*,* ist eine prospektive Machbarkeitsstudie mit dem FUSION-X-US-Prototyp zur Durchführung der digitalen Brusttomosynthese und der ABVS bei 23 Patientinnen bekannt, bei denen nach der klinischen Untersuchung und der Standardbildgebung eine Indikation zur Tomosynthese gemäß den aktuellen Leitlinien bestand. Die ABVS- und Tomosynthese-Bilder des Prototyps wurden von zwei Experten getrennt interpretiert. Die Studie vergleicht die Erkennung und die BT-RADS(E)-Scores von Brustläsionen unter ausschließlicher Verwendung der Tomosynthese- und ABVS-Daten des FUSIONX-US-Prototyps mit den Ergebnissen der vollständigen diagnostischen Abklärung.

Aus der Druckschrift EP 3 219 261 A1 ist ein Verfahren zur Brustbildrekonstruktion bekannt, welches das Positionieren einer Brust auf einer Trägerplatte eines Bildgebungssystems, das Zusammendrücken der Brust mit einem flexiblen Paddel, das Erhalten von Bildgebungsdaten, und das Schätzen eines Brustdickenprofils durch mindestens eines der folgenden Verfahren umfasst: Platzieren von Markierungen auf der Brust, Durchführen einer bildbasierten Analyse der erhaltenen Daten, Verwenden eines Hilfssystems und Durchführen einer modellbasierten Berechnung. Die dreidimensionale Rekonstruktion umfasst die Verwendung eines Dickenprofils der Brustoberfläche in mindestens einer von einer iterativen Rekonstruktion, einer gefilterten Rückprojektionsrekonstruktion und einer gemeinsamen Rekonstruktion, die unter Verwendung von aus einem Ultraschallscan erhaltenen Informationen durchgeführt wird.

Es ist daher eine Aufgabe der vorliegenden Erfindung, eine örtliche Zuordnung zwischen einem zweidimensionalen Röntgenprojektionsbild und einem Ultraschall-Volumendatensatz anzugeben und bei möglichst geringer Strahlendosis eine gute Befundung zu ermöglichen.

Diese Aufgabe wird durch ein Verfahren zur Ermittlung eines interessierenden Bereichs gemäß Patentanspruch 1, ein Verfahren zur Darstellung eines interessierenden Bereichs gemäß Patentanspruch 10 eine Bildverarbeitungseinrichtung gemäß Patentanspruch 11 und ein Röntgensystem gemäß Patentanspruch 12 gelöst.

Das zuvor genannte Verfahren zur Ermittlung eines darzustellenden interessierenden Bereichs (ROI) in einem Ultraschall-Volumendatensatz vom Inneren eines Untersuchungsobjekts, der mittels eines Röntgen-3D-Ultraschall-Geräts akquiriert wurde, umfasst zumindest die Schritte gemäss Anspruch 1.

Das Untersuchungsobjekt kann grundsätzlich jedes mittels Röntgen und Ultraschall zu untersuchende Objekt sein. Bevorzugt handelt es sich jedoch um die Brust einer menschlichen, weiblichen Patientin. Bei dem darzustellenden interessierenden Bereich handelt es sich um einen Bereich, der im Rahmen einer Untersuchung bzw. Befundung besonders zu berücksichtigen ist. Dabei kann es sich beispielsweise Läsionen, Mikrokalzifikationen, Knoten oder dergleichen handeln.

Die Daten, also das Röntgenprojektionsbild und der Ultraschall-Volumendatensatz, werden vor Beginn des eigentlichen erfindungsgemäßen Verfahrens akquiriert, insbesondere mit Hilfe eines Röntgen-3D-Ultraschall-Gerät (XUS-Gerät). Dabei handelt es sich um ein Kombinationsgerät, mit dem sowohl Röntgenbilder als auch Ultraschallaufnahmen des Untersuchungsobjekts erzeugt werden können. Es weist also bevorzugt eine Röntgenquelle sowie ein Röntgendetektor auf und umfasst zugleich eine Ultraschallsonde, die ausgebildet ist um Ultraschallwellen zu emittieren und deren Reflexionen am Untersuchungsobjekt zu detektieren. Bevorzugt handelt es sich bei dem XUS-Gerät entsprechend dem Untersuchungsobjekt also um ein kombiniertes Ultraschall-Mammographiegerät.

Mit dem XUS-Gerät können beispielsweise in einem vorbereitenden Schritt vor dem erfindungsgemäßen Verfahren sowohl das zweidimensionalen Röntgenprojektionsbild als auch der dreidimensionale Ultraschall-Volumendatensatz akquiriert werden.

Das Röntgenprojektionsbild und/oder der Ultraschall-Volumendatensatz können aber zum Beispiel auch in einem Speicher hinterlegt sein und aus diesem zur Durchführung des erfindungsgemäßen Verfahrens abgerufen werden. Bei dem Röntgenprojektionsbild handelt es sich bevorzugt um ein Mammogramm. Als Projektionsposition wird grundsätzlich eine Position im Röntgenprojektionsbild verstanden, also beispielsweise ein Bildpunkt, der bevorzugt einem Pixel eines Röntgendetektors entspricht. Die Projektionsposition liegt dabei bevorzugt, besonders bevorzugt möglichst zentral, im interessierenden Bereich. Die Ermittlung kann dabei durch einen Eingabe eines Benutzers und/oder mittels eines Computeralgorithmus ("computer aided detection" - CAD) erfolgen, wie später noch näher erläutert wird. Die Projektionsposition wird insbesondere nur in einem bzw. in genau einem Röntgenprojektionsbild ermittelt, da auf diese Art die Strahlenbelastung für den Patienten vorteilhaft gering gehalten werden kann.

Der Projektionsstrahl bezeichnet eine Verbindungslinie zwischen der Projektionsposition bzw. einem ihr zugeordneten Detektorpixel und der Röntgenquelle, d. h. dem Röntgenfokus. Die Röntgenquelle weist dabei insbesondere eine kegelförmige Strahlengeometrie auf. Dadurch kommt es insbesondere in Randbereichen des Detektors zu schrägen, d. h. vom senkrechten Einfall abweichenden, Einfallswinkeln. Unter Vernachlässigung von Streustrahleneffekten gibt das Pixel bzw. der Bildpunkt an der Projektionsposition als Bildpunktwert bzw. Helligkeit also eine aufsummierte bzw. integrierte Information über das Gewebe des interessierenden Bereichs wieder, das entlang des Projektionsstrahls durchdrungen wurde.

Um diese Information vollständig auch im dreidimensionalen Ultraschall-Volumendatensatz visualisieren zu können, ohne den gesamten Ultraschall-Volumendatensatz heranzuziehen, wird ein Teil der Daten ausgewählt und daraus ein Teildatensatz erzeugt, d. h. extrahiert. Dies wird anhand der Projektionsposition ausgeführt. D. h. die Auswahl der Daten erfolgt gemäß einer Zuordnung bzw. einer Korrelation zwischen der Projektionsposition und zumindest einem Teil der Daten des Ultraschall-Volumendatensatzes, welche der Projektionsposition entsprechen d. h. Informationen über dieselben Bereiche des Untersuchungsobjekts wie auch der Bildpunkt der Projektionsposition umfassen. Der Teildatensatz was dabei bevorzugt einen kleinen Teil der Daten des Ultraschall-Volumendatensatzes, also beispielsweise weniger als 20 %, bevorzugt weniger als 10 %, damit sich ein Befunder möglichst schnell ein detailliertes Bild von den interessierenden Bereich schaffen kann.

Zudem werden bei der Extraktion des Teildatensatzes geometrische Informationen bzw. Lageinformationen betreffend das Mammographie-3D-Ultraschall-Gerät herangezogen. Dabei handelt es sich insbesondere um eine relative Lage, d. h. Position in den translatorischen Freiheitsgraden und/oder Orientierung in den rotatorischen Freiheitsgraden, zwischen der Röntgenquelle und Detektor, also auch zwischen der Röntgenquelle und dem Pixel des Detektors bzw. dem Bildpunkt im Röntgenprojektionsbild. Mit Hilfe dieser Information kann der Weg eines Projektionsstrahls von der Röntgenquelle bis zum Detektor nachvollzogen werden. Dadurch kann die Zuordnung der Bereiche des Untersuchungsobjekts, die vom Strahl durchdrungen wurden, bzw. den entsprechenden Daten des Ultraschall-Volumendatensatzes und der Projektionsposition getroffen werden. Unter geometrischen Informationen können allerdings noch weitere Informationen gefasst sein, wie später noch näher erläutert wird.

Das erfindungsgemäße Verfahren kann für jeden interessierenden Bereich des Untersuchungsobjekts wiederholt werden, sodass alle interessierenden Bereiche des Untersuchungsobjekts in einfacher Weise betrachtet bzw. befundet werden können.

Bei dem eingangs genannten Verfahren zur Darstellung eines interessierenden Bereich wird eine Anzahl von Ansichten aus einem Teildatensatz, der gemäß eines erfindungsgemäßen Verfahrens zur Ermittlung eines darzustellenden Bereiches ermittelt wurde, bereitgestellt und zumindest eine Ansicht dargestellt.

Bei der Anzahl von Ansichten kann es sich um eine oder mehrere Ansichten handeln, die eine (pseudo-)dreidimensionale, bevorzugt zweidimensionale, Darstellung des Teildatensatzes wiedergeben. Die Anzahl der Ansichten Ansicht kann dabei also beispielsweise ein einzelnes zweidimensionales Bild oder einen mehrere Bilder aufweisenden Schichtbildstapel umfassen.

Die Bereitstellung der Ansichten kann dabei zeitlich unabhängig von deren Darstellung erfolgen, indem die Ansichten beispielsweise in einem Speicher hinterlegt werden. Unabhängig davon ob sie direkt generiert oder aus einem Speicher abgerufen werden, kann ihre Darstellung mithilfe von einem üblichen Anzeigegerät, wie beispielsweise einem Monitor, einem sonstigen Display, einer 3D Brille, einem Beamer bzw. Projektor oder dergleichen erfolgen.

Die eingangs genannte Bildverarbeitungseinrichtung umfasst alle Komponenten zur Ausführung eines der oben beschriebenen erfindungsgemäßen Verfahren.

Grundsätzlich kann die Bildverarbeitungseinrichtung unabhängig von den für die Akquisition benötigten Modalitäten arbeiten. Mit ihr können also z. B. auch Daten von separaten Röntgen- und Ultraschallgeräten erfindungsgemäß verarbeitet werden. So kann beispielsweise die Lage eines Schallkopfes des Ultraschallgeräts relativ zum Untersuchungsobjekt und/oder Röntgengerät sensorisch mittels bekannter Verfahren ermittelt bzw. getrackt werden. Dadurch kann auch die relative Lage von dem Röntgenprojektionsbild und dem dreidimensionalen Ultraschalldatensatz ermittelt werden.

Das eingangs genannte Röntgensystem weist eine erfindungsgemäße Bildverarbeitungseinrichtung und ein Röntgen-3D-Ultraschall-Gerät auf. Letzteres umfasst eine Quelle-Detektor-Anordnung, die zur Aufnahme eines Röntgenprojektionsbilds eines Untersuchungsobjekts ausgebildet ist. Zudem umfasst das XUS-Gerät eine Ultraschallanordnung, die zur Aufnahme eines Ultraschall-Volumendatensatzes ausgebildet ist. Bei dem XUS-Gerät handelt es sich bevorzugt um ein Mammographiegerät, das im Vergleich zu üblichen Mammographiegeräten zusätzlich eine Ultraschallanordnung aufweist.

Die erfindungsgemäße Bildverarbeitungseinrichtung kann vorteilhafterweise in bereits existenten Röntgensystemen nachgerüstet werden. Ebenso ist es jedoch möglich neu zu fertigende Röntgensysteme bereits bei der Fertigung mit einer erfindungsgemäßen Bildverarbeitungseinrichtung auszustatten.

Die wesentlichen Komponenten der erfindungsgemäßen Bildverarbeitungseinrichtung können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Insbesondere kann die erfindungsgemäße Bildverarbeitungseinrichtung Teil eines Benutzerterminals eines Röntgensystems sein.

Eine weitgehend softwaremäßige Realisierung hat den Vorteil, dass auch schon bisher verwendete Bildverarbeitungseinrichtung auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Bildverarbeitungseinrichtung eines Röntgensystems ladbar ist, mit Programmabschnitten, um alle Schritte des erfindungsgemäßen Verfahrens auszuführen, wenn das Programm in der Bildverarbeitungseinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Bildverarbeitungseinrichtung und/oder zur Speicherung an oder in der Bildverarbeitungseinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Bildverarbeitungseinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen oder Beschreibungsteilen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Grundsätzlich ist es möglich den darzustellenden Bereich einzugrenzen, indem beispielsweise Randbereiche ausgegrenzt werden, die sich in der Nähe der Brustwand befinden. Bevorzugt umfasst der Teildatensatz, insbesondere zumindest alle, die Teildaten, die Bereichen des Untersuchungsobjekts zugeordnet werden, die vom Projektionsstrahl durchdrungen wurden. Der Teildatensatz umfasst also insbesondere die gesamte Information, die auch in dem Bildpunkt der Projektionsposition akkumuliert ist. D.h. es sind zumindest die Bereiche bzw. Teile des Ultraschall-Volumendatensatzes enthalten, die einer Verbindungslinie des entsprechenden Detektorpixels zu der Röntgenquelle zugeordnet werden. Dadurch wird sichergestellt, dass der Befunder auch im Teildatensatz zumindest die Formation vorfindet, die er aufgrund der ermittelten Projektionsposition erwartet. Zudem kann der Teildatensatz noch weitere Daten, insbesondere aus dem interessierenden Bereich, umfassen, wie im Folgenden noch näher erläutert wird.

In einer ersten Variante ist der Teildatensatz bevorzugt als eine Fläche ausgebildet. Die Fläche ergibt sich dabei beispielsweise als Schnittmenge des Ultraschall-Volumendatensatzes mit einer Ebene, die im mathematischen Sinne ein unbegrenzt ausgedehntes zweidimensionales Objekt darstellt. Die Ebene ist beispielsweise mithilfe von drei Vektoren bzw. der Dreipunkteform definiert. Ein erster Vektor bzw. ein Stützvektor kann dabei als Ursprungsvektor frei festgelegt werden und ist daher zum Beispiel in einem Punkt (Ursprung des definierten Koordinatensystems) einer senkrechten Projektion der Röntgenquelle auf den Detektor gegeben. Die Ebene kann weiterhin beispielsweise durch folgende zwei Stützvektoren aufgespannt sein:
- ein erster Richtungsvektor (U bzw. z) senkrecht zur Detektorfläche, d.h., in Richtung vom Ursprung zu der Röntgenquelle (oder umgekehrt) und/oder ein erster Richtungsvektor (U bzw. z) in einer Ausbreitungsrichtung, also in einer Hauptausbreitungsrichtung, der Ultraschallwellen,
- ein zweiter Richtungsvektor (V) in Richtung vom Ursprung zu der Projektionsposition.

Dabei ist die Wahl des ersten Richtungsvektors in der Ausbreitungsrichtung der Ultraschallwellen besonders vorteilhaft, da so Abschattungseffekte, also z. B. die Schallabschattung, die durch eine Läsion erzeugt wird, gut erkennbar sind.

Aufgrund der geometrischen Konstruktion liegt automatisch auch der Projektionsstrahl vollständig in der zuvor beschriebenen Ebene. Durch einen Schnitt der Ebene mit dem Ultraschall-Volumendatensatz wird vorteilhafterweise ein zweidimensionaler Teildatensatz definiert bzw. extrahiert der auch all die entsprechenden Informationen bezüglich der Bereiche des Untersuchungsobjekts enthält, die vom Projektionsstrahl durchdrungen wurden. Diese Fläche kann beispielsweise im Rahmen des eingangs beschriebenen Verfahrens zur Darstellung einfach als zweidimensionale Ansicht bzw. Bild bereitgestellt und dargestellt werden.

In einer zweiten Variante umfasst der Teildatensatz bevorzugt Daten aus einem definierten Volumenbereich.

In einfacher Weise kann der Volumenbereich als der Bereich festgelegt sein, der zur zugehörigen Projektionsposition eine Entfernung von höchstens 2 cm, bevorzugt höchstens 1 cm, in einer Richtung senkrecht zur Hauptausbreitungsrichtung der Ultraschallwellen aufweist.

Der Volumenbereich umfasst bevorzugt einen Korridor durch das Untersuchungsobjekt, der sich in einer ersten Richtung des Projektionsstrahls erstreckt und eine Korridorbreite von höchstens 2 cm, besonders bevorzugt höchstens 1 cm, aufweist. Dass sich der Korridor in einer ersten Richtung des Projektionsstrahls erstreckt heißt, dass seine Haupterstreckungsrichtung in dieser Richtung angeordnet ist. Bei der ersten Richtung des Projektionsstrahls handelt es sich bevorzugt um dessen Komponente senkrecht zu einer Hauptausbreitungsrichtung der Ultraschallwellen. Besonders bevorzugt ist diese Komponente zugleich parallel zu der Detektorfläche. Die Korridorbreite gibt dabei die Ausdehnung des Korridors in einer Richtung senkrecht zur Haupterstreckungsrichtung an, die bevorzugt zugleich parallel zur Detektorfläche und/oder senkrecht zur Hauptausbreitungsrichtung der Ultraschallwellen.

Der Volumenbereich umfasst alternativ bevorzugt einen Winkelbereich. Der Scheitelpunkt des Winkelbereichs ist dabei besonders bevorzugt auf der Position der Röntgenquelle angeordnet. Bei einer kegelförmigen Geometrie des Winkelbereichs bzw. des von der Röntgenquelle ausgesandten Strahlengangs ergibt sich zum Beispiel als Schnittmenge zwischen dem Winkelbereich und dem Ultraschall-Volumendatensatz ein Kegelabschnitt.

Es wird also ein dreidimensionaler Teildatensatz erzeugt, der zumindest auch die Bereiche umfasst, die dem Projektionsstrahl zugeordnet sind. Dabei wird der vom Teildatensatz umfasste Bereich in Randbereichen des Detektors größer, d.h. größer je weiter die Projektionsposition von der Röntgenquelle entfernt ist.

Besonders bevorzugt umfasst der Teildatensatz jedoch Daten eines quaderförmigen Bereichs (Bounding-Box) des Ultraschall-Volumendatensatzes, der den zuvor beschriebenen Winkelbereich, insbesondere gerade genau, vollständig umfasst. Dadurch wird vorteilhafterweise die folgende Darstellung vereinfacht.

Der Teildatensatz kann wie oben beschrieben beispielsweise als seriell, d. h. aufeinanderfolgende, ihrer Position nach geordnete Schichtbilder eines Schichtbildstapels dargestellt werden. Dabei können die Schildbilder jeweils beispielsweise parallel zur Detektorfläche oder z. B. senkrecht zum Projektionsstrahl angeordnet sein. Insbesondere entsprechen sich die Position bzw. Tiefe im Untersuchungsobjekt und die Position im Schichtbildstapel.

Vorzugsweise bildet der Projektionsstrahl eine Mittelachse des Winkelbereichs. D. h. der Winkelbereich ist so angeordnet, dass sich der Projektionsstrahl jeweils durch den Mittelpunkt einer Schnittfläche mit dem Winkelbereichs erstreckt, die zu ihm senkrecht ist. Durch eine derart zentrale Anordnung des Projektionsstrahls Teildatensatz wird sichergestellt, dass der Befunder auch Bereiche in die Befundung mit einbeziehen kann, die sich angrenzend zu den dem Projektionsstrahl zugeordneten Bereichen befinden.

Der Winkelbereich erstreckt sich bevorzugt in Winkeln von höchstens 15°, besonders bevorzugt höchstens 10°, ganz besonders bevorzugt höchstens 5°, zum Projektionsstrahl erstreckt. Dadurch können insbesondere die Randbereiche des Detektors, in denen die Strahlungsintensität und somit die Diagnosequalität im Röntgenprojektionsbild geringer ist, größer flächig im Ultraschall-Volumendatensatz bzw. Teildatensatz untersucht werden. Damit wird auch in diesen Bereichen eine gute Befundung sichergestellt.

Der Teildatensatz umfasst vorzugsweise Teildaten, die einem Volumen von höchstens 3 cm³, besonders bevorzugt höchstens 2 cm³, ganz besonders bevorzugt höchstens 1 cm³, entsprechen. Durch diese Beschränkung des Teildatensatzes sichergestellt, dass der Befunder keine großen Schichtbild stapeln beurteilen muss. Dadurch kann er sich vorteilhafterweise auf die Details in dem interessierenden Bereich konzentrieren.

Der Teildatensatz wird bevorzugt mittels einer automatischen Detektion und/oder einer automatischen Klassifizierung des interessierenden Bereichs analysiert. Dazu kann der Teildatensatz z. B. an ein integriertes oder auch an ein externes CAD-System übermittelt. Mittels bekannter CAD-Verfahren wird dann eine exakte Position des interessierenden Bereichs ermittelt, der interessierende Bereich wird also lokalisiert. Alternativ oder zusätzlich erfolgt eine Klassifizierung mittels bekannter CAD-Verfahren. Es wird also beispielsweise ermittelt, ob es sich um eine gutartige oder eine bösartige Gewebeveränderung handelt. Je nach Bedarf kann ggf. auch eine genauere Klassifizierung bzw. Klassifizierung der Gewebeveränderung erfolgen.

Bevorzugt wurden der Ultraschall-Volumendatensatz und das zweidimensionale Röntgenprojektionsbild während eines, insbesondere eines einzigen, Kompressionsschritts des Untersuchungsobjekts akquiriert. Dadurch kann eine umständliche Registrierung der akquirierten Daten aufeinander vermieden werden. Damit wird zudem eine Zeitersparnis bei der Untersuchung erreicht wird und zugleich werden Unannehmlichkeiten für den Patienten verringert sowie Personal- und Gerätekosten eingespart.

Die geometrischen Informationen umfassen bevorzugt zumindest eine der folgenden Informationen: Strahlgeometrie, Abstand zwischen Röntgenquelle und Detektor, Dicke des Untersuchungsobjekts, Abstand zwischen Röntgenquelle und Projektionsposition.

Die Strahlgeometrie beschreibt die geometrischen Eigenschaften des Strahlengangs der Röntgenquelle. Dabei sind unter anderem parallele Strahlengeometrien und kegelförmigen Strahlengeometrien bekannt, wobei aufgrund der einfacheren technischen Umsetzbarkeit kegelförmige Strahlengeometrien am häufigsten in der Praxis eingesetzt werden. Zur weiteren Charakterisierung einer kegelförmigen Strahlengeometrie wird unter anderem der Abstrahlwinkel bzw. Blendwinkel herangezogen.

Der Abstand zwischen der Röntgenquelle und dem Detektor beschreibt die kürzeste Entfernung zwischen den beiden Komponenten. Aus der Praxis sind Röntgengeräte bekannt, bei denen dieser Abstand z. B. d mittels einer Verstelleinrichtung variabel einstellbar ist. In diesen Fällen muss der Abstand zwischen der Röntgenquelle und dem Detektor erfasst werden. Dies kann beispielsweise bereits im Rahmen der Regelung der Verstelleinrichtung, also z. B. über eine interne Positionsbestimmung und/oder über einen eingebauten Schrittmotor, und/oder mittels einem zusätzlichen Abstands- bzw. Entfernungsmesser, wie z. B. einem Laserentfernungsmesser, erfolgen kann.

Auch die Dicke des Untersuchungsobjekts entspricht einer geometrischen Information betreffend das XUS-Gerät. Sie beschreibt die Ausbreitung des Untersuchungsobjekts in einer Richtung senkrecht zu der Detektorfläche. Die Dicke des Untersuchungsobjekts kann beispielsweise mittels des Abstands zwischen dem Detektor und einer darauf herabsenkbaren, d.h. zum Detektor hin und von ihm weg bewegbaren, Kompressionsplatte ermittelt werden. Dieser Abstand kann mithilfe der oben bereits beschriebenen Mittel erfasst werden. Alternativ oder zusätzlich kann die Dicke des Untersuchungsobjekts auch aus dem Ultraschall-Volumendatensatz ermittelt werden. Bei bekannter Lage des Projektionsstrahls können mit ihrer Hilfe ein Eintrittspunkt in das bzw. ein Austrittspunkt aus dem Untersuchungsobjekt und folglich auch die im Untersuchungsobjekt durchdrungenen Bereiche ermittelt werden.

Der Abstand zwischen der Röntgenquelle und der Projektionsposition entspricht, wie oben bereits beschrieben, dem Abstand zwischen der Röntgenquelle und dem Detektorpixeln, das dem Bildpunkt der Projektionsposition zugeordnet ist. Dieser Abstand kann in einfacher Weise ermittelt werden, wenn der Abstand der Röntgenquelle zu dem Detektor und die Abmessungen des Detektors selbst bekannt sind. Besonders bevorzugt wird die relative Lage der Projektionsposition zur Röntgenquelle ermittelt. Dadurch lässt sich der Verlauf des Projektionsstrahls als Verbindungslinie zwischen der Projektionsposition bzw. dem ansprechenden Detektorpixel und der Röntgenquelle rekonstruieren.

Das Ermitteln der Projektionsposition erfolgt automatisch. Die Produktionsposition kann als Eingabe durch einen Bediener bzw. Befunder erfolgen, indem er zum Beispiel in dem angezeigten Röntgenprojektionsbild auf einen entsprechenden Bildpunkt klickt, Bildpunktkoordinaten eingeht oder dergleichen, eine solche Eingabe wird jedoch nicht beansprucht. Die Projektionsposition wird automatisch mittels eines CAD-Algorithmus (computer aided dectection) ermittelt. Aus der Praxis ist eine Vielzahl solcher Algorithmen bekannt die beispielsweise Methoden wie eine Schwellenwertanalyse, eine Kantendetektion und/oder der gleichen verwenden.

In Kombination kann die Ermittlung beispielsweise erfolgen, indem durch den Bediener ein Bereich eingegeben wird, in dem die automatische Ermittlung erfolgen soll. Umgekehrt kann im Rahmen eines CAD-Algorithmus zunächst ein Bereich ermittelt werden, in dem durch den Bediener die Projektionsposition angegeben wird. Ferner ist es auch möglich, mittels des CAD-Algorithmus eine Projektionsposition vorzuschlagen, die dann von dem Bediener gegebenenfalls korrigiert werden kann. Eine solche Kombination wird jedoch nicht beansprucht.

Insgesamt ermöglicht es die Erfindung, einen verhältnismäßig großen Ultraschall-Volumendatensatz für die Befundung auf einen kleinen Teildatensatz zu reduzieren, indem das Röntgenprojektionsbild als Übersicht bzw. zur Auswahl des interessierenden Bereichs bzw. der Projektionsposition genutzt wird. Dadurch wird eine schnellere und trotzdem gründliche Befundung ermöglicht, wobei der Befunder lediglich mit solchen Arten von Bildmaterial konfrontiert wird, die ihm aus seiner täglichen Berufspraxis bereits bekannt sind. Zudem wird die Röntgendosis für den Patienten vorteilhaft gering gehalten, da lediglich ein Röntgenprojektionsbild als Übersichtsaufnahme angefertigt werden muss.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
- FIG 1: eine grob schematische Darstellung eines Ausführungsbeispiels eines erfindungsgemäßen Röntgensystems,
- FIG 2: ein schematisches Blockschaltbild eines Ausführungsbeispiels einer erfindungsgemäßen Bildverarbeitungseinrichtung mit einer Darstellungseinrichtung,
- FIG 3: ein schematisches Blockdiagramm für den Ablauf eines Ausführungsbeispiels eines erfindungsgemäßen Verfahrens zur Ermittlung und Darstellung eines interessierenden Bereichs,
- FIG 4: eine schematische perspektivische Darstellung der Extraktion eines Teildatensatzes gemäß einem ersten Ausführungsbeispiel eines erfindungsgemäßen Verfahrens zur Ermittlung eines darzustellenden interessierenden Bereichs,
- FIG 5: eine schematische perspektivische Darstellung der Extraktion eines Teildatensatzes gemäß einem zweiten Ausführungsbeispiel eines erfindungsgemäßen Verfahrens zur Ermittlung eines darzustellenden interessierenden Bereichs und
- FIG 6: eine Ansicht, die mittels eines Ausführungsbeispiels eines erfindungsgemäßen Verfahrens zur Darstellung eines interessierenden Bereich aus einem Ultraschall-Volumendatensatz ermittelt wurde.

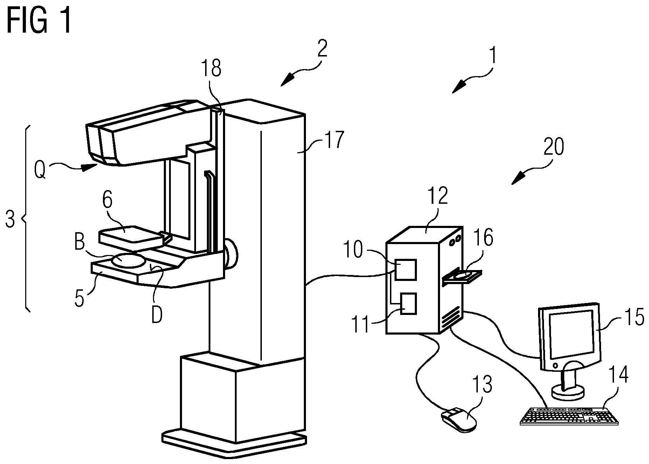

In FIG 1 ist beispielhaft und grob schematisch ein erfindungsgemäßes Röntgensystem 1, hier ein Mammographiesystem, gezeigt. Relative Richtungsangaben wie "oben" und "unten" etc. beziehen sich auf ein bestimmungsgemäß für den Betrieb aufgestelltes Mammographiesystem 1. Das Mammographiesystem 1 umfasst ein kombiniertes Röntgen-Ultraschall-Gerät 2 (XUS-Gerät), hier ein Mammographie-Ultraschall-Gerät, und ein Rechnersystem 20. Das XUS-Gerät 2 weist eine Standsäule 17 und Quelle-Detektor-Anordnung 3 auf, die wiederum eine Röntgenquelle Q und einen Detektor 5 mit einer Detektionsfläche D umfasst. Die Standsäule 17 steht im Betrieb auf dem Untergrund. Mit ihr ist die Quelle-Detektor-Anordnung 3 verschiebbar verbunden, sodass die Höhe der Detektorfläche D, also der Abstand zum Untergrund, auf eine Brusthöhe einer Patientin eingestellt werden kann.

Eine Brust B der Patientin (hier schematisch dargestellt) liegt als Untersuchungsobjekt B für eine Untersuchung oberseitig auf der Detektorfläche D auf. Über der Brust B und der Detektorfläche D ist eine Ultraschallanordnung 6 angeordnet, die zugleich als Kompressionsplatte dient und verschiebbar mit der Quelle-Detektor-Anordnung 3 verbunden ist. Für die Untersuchung wird die Brust B komprimiert und zugleich fixiert, indem die Ultraschallanordnung 6 auf sie herabgesenkt wird, sodass auf die Brust O zwischen Ultraschallanordnung 6 und Detektorfläche D ein Druck ausgeübt wird.

Die Röntgenstrahlungsquelle Q ist dem Detektor 5 gegenüberliegend so angeordnet und ausgebildet, dass der Detektor 5 von ihr emittierte Röntgenstrahlung erfasst, nachdem zumindest ein Teil der Röntgenstrahlung die Brust O der Patientin durchdrungen hat. Es wird also ein Röntgenprojektionsbild erfasst. Dabei ist die Quelle-Detektor-Anordnung 3 relativ zur Standsäule 17 mittels eines Dreharms 18 in einem Bereich von beispielsweise ± 90 um eine Grundstellung schwenkbar, in der sie senkrecht zum Untergrund steht. Mittels einer Schwenkung der Quelle-Detektor-Anordnung 3 können Daten der Brust B aus unterschiedlichen Körperrichtungen der Patientin akquiriert werden, also z. B. cranio-caudal, medio-lateral, medio-lateral-oblique oder dergleichen.

Die Ultraschallanordnung 6 umfasst eine Ultraschallsonde (hier nicht gezeigt), die im Betrieb Ultraschallwellen in Richtung der Brust B aussendet. Die Ultraschallwellen werden an den unterschiedlichen Strukturen der Brust B gestreut, gebrochen und reflektiert. Die zurückkommenden Ultraschallwellen werden von der Ultraschallsonde wieder empfangen und anschließend ausgewertet und umgewandelt, so dass ein dreidimensionaler Ultraschall-Volumendatensatz 3D-US vom Inneren der Brust B erzeugt werden kann.

Das Rechnersystem 20 umfasst eine Rechnereinheit 12 und jeweils damit verbunden eine Maus 13, eine Tastatur 14 sowie einen Bildschirm 15. Der Bildschirm 15 dient hier als Anzeigeeinheit 15, Maus 13 und Tastatur 14 dienen jeweils als Eingabegerät. Die Rechnereinheit 12 umfasst eine Bildverarbeitungseinrichtung 10 und eine Darstellungseinrichtung 11 (hier schematisch als Blöcke dargestellt) sowie ein Laufwerk 16 zum Einlesen von CD bzw. DVD. Dabei können die Bildverarbeitungseinrichtung 10 und die Darstellungseinrichtung 11 gemeinsam Komponenten der Rechnereinheit 12 nutzen, wie z. B. Speicher, Prozessoren und dergleichen. Das Rechnersystem 20 kann in demselben Raum wie das XUS-Gerät 2 angeordnet sein, es kann sich aber auch in einem angrenzenden Kontrollraum oder in einer noch weiteren räumlichen Entfernung befinden.

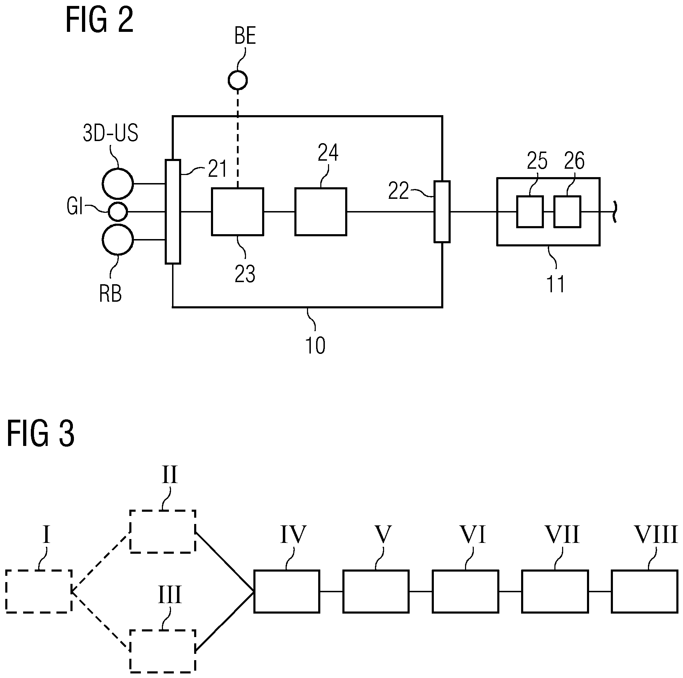

In FIG 2 ist ein Ausführungsbeispiel einer erfindungsgemäßen Bildverarbeitungseinrichtung 10 zusammen mit einem Ausführungsbeispiel einer Darstellungseinrichtung 11 gezeigt. Die Bildverarbeitungseinrichtung 10 umfasst eine Eingangsschnittstelle 21 und eine Ausgangsschnittstelle 22 sowie eine Ermittlungseinheit 23 und eine Extraktionseinheit 24. Die Eingangsschnittstelle 21 ist mit der Ermittlungseinheit 23 verbunden und übermittelt der Ermittlungseinheit 23 eingehende Daten, die einen dreidimensionalen Ultraschall-Volumendatensatz 3D-US, ein Röntgenprojektionsbild RB und geometrische Informationen GI umfassen.

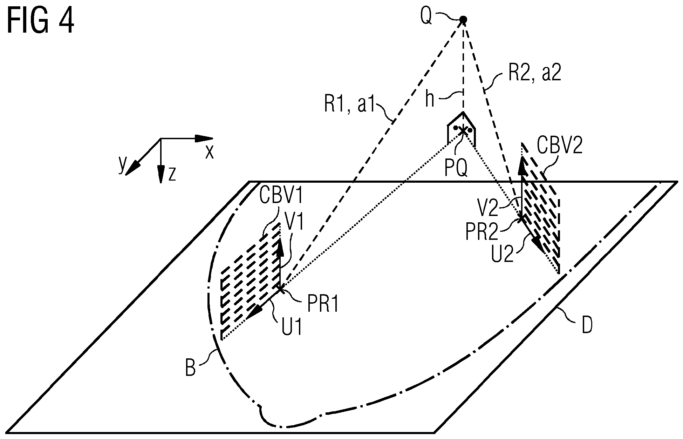

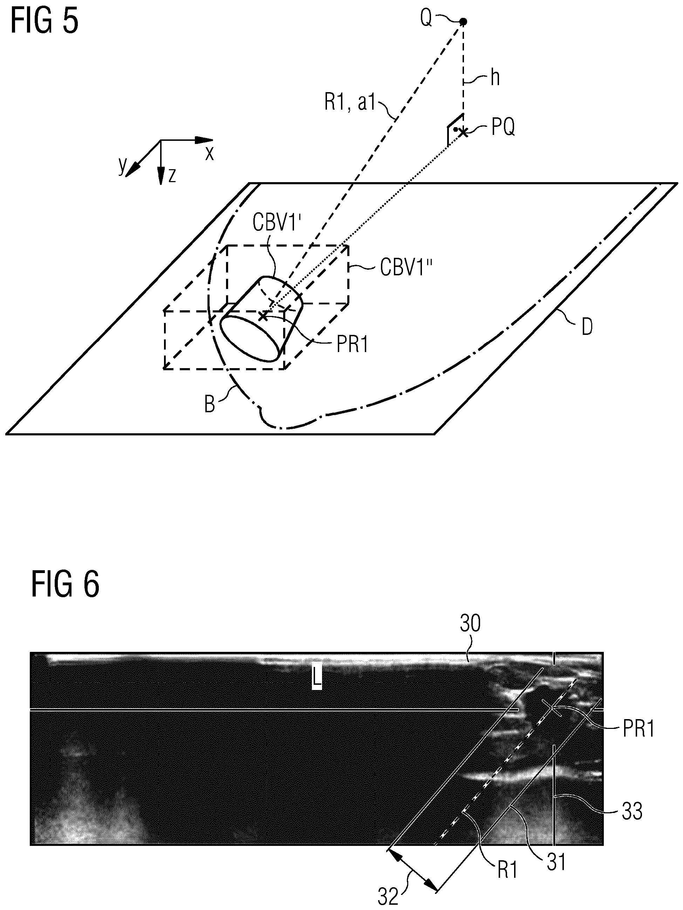

Aus dem Röntgenprojektionsbild RB wird beispielsweise mittels einer Benutzereingabe BE eine Projektionsposition PR1, PR2 (siehe FIG 4, FIG5) ermittelt. Die ermittelte Projektionsposition PR1, PR2 sowie der Ultraschall-Volumendatensatz 3D-US und die geometrische Information GI werden an die Extraktionseinheit 24 übermittelt. Dort wird auf Basis der Projektionsposition PR1, PR2 und der geometrischen Informationen GI aus dem Ultraschall-Volumendatensatz 3D-US ein Teildatensatz CBV1, CBV1', CBV2 extrahiert, wie anhand von FIG3 bis FIG 5 noch detaillierter erläutert wird. Der Teildatensatz CBV1, CBV1', CBV2 wird über die Ausgangsschnittstelle 22 an die Darstellungseinrichtung 11 weitergeleitet.

Die Darstellungseinrichtung 11 umfasst eine Bereitstellungseinheit 25 und eine Darstellungseinheit 26. Die Bereitstellungseinheit 25 wählt aus dem Teildatensatz CBV1, CBV1', CBV2 eine Anzahl von Ansichten aus, in denen der interessierende Bereich für einen Befunder möglichst günstig dargestellt werden kann. Diese Ansichten werden mittels der Darstellungseinheit 26 so aufbereitet, dass sie von einer üblichen Anzeigeeinheit wie beispielsweise dem Bildschirm 15 dargestellt werden können.

FIG3 zeigt grob schematisch ein Blockdiagramm, das in zwei Schritten IV und V den Ablauf eines Ausführungsbeispiels eines erfindungsgemäßen Verfahrens zur Ermittlung eines darzustellenden interessierenden Bereichs sowie in zwei Schritten VI und VII den Ablauf eines Ausführungsbeispiels eines erfindungsgemäßen Verfahrens zur Darstellung eines interessierenden Bereichs darstellt.

In einem vorbereitenden ersten Schritt I, der nicht vom erfindungsgemäßen Verfahren umfasst ist, wird die Brust B der Patientin zwischen den Detektor 5 und der Ultraschallanordnung 6 komprimiert, indem die Ultraschallanordnung 6 auf den Detektor 5 herabgesenkt wird, bis ein vordefinierter Druck auf die Brust B ausgeübt wird. Im komprimierten Zustand ist die Brust B sowohl mittels Röntgenstrahlung als auch mittels Ultraschall besser zu untersuchen und zudem so fixiert, dass zwischen den einzelnen im Folgenden beschriebenen Akquisitionsschritten keine und Positionierung der Brust B erfolgt.

In einem vorbereitenden zweiten Schritt II, der ebenfalls nicht vom erfindungsgemäßen Verfahren umfasst ist, erfolgt die Akquisition eines Röntgenprojektionsbildes RB. Wie eingangs bereits beschrieben, wird mittels Röntgenquelle Q Röntgenstrahlung erzeugt die Brust B durchdringt und dann geschwächt auf die Detektorfläche D auftrifft und in Abhängigkeit von ihrer Intensität detektiert wird. Die Schwächung bzw. Absorption, also die Verringerung der detektierten Intensität, der Röntgenstrahlung ist dabei abhängig von den Strukturen bzw. der Dichte des bestrahlten Gewebes der Brust B. Das akquirierte Röntgenprojektionsbildes RB kann beispielsweise auf dem Bildschirm 15 angezeigt werden.

In einem vorbereitenden Schritt III der ebenfalls nicht vom erfindungsgemäßen Verfahren umfasst ist, erfolgt die Akquisition eines dreidimensionalen Ultraschall-Volumendatensatzes 3D-US. Dazu werden nach einem dem Fachmann bekannten Verfahren Ultraschallwellen in Form von Ultraschallpulsen unterschiedlichen Positionen in Bezug auf die Brust B nach Art eines Scans ausgesendet. Diese werden an den unterschiedlichen Strukturen des zu untersuchenden Objekts gestreut, gebrochen und reflektiert. Die zurückkommenden Ultraschallwellen werden von der Ultraschallsonde wieder empfangen und anschließend ausgewertet und in den dreidimensionalen Ultraschall-Volumendatensatz 3D-US umgewandelt.

Die Schritte II und III können grundsätzlich zeitlich unabhängig voneinander ausgeführt werden, solange die Transmission der Röntgenstrahlung durch die Ultraschallanordnung 6 gewährleistet ist. Bevorzugt erfolgt die Akquisition des Röntgenprojektionsbildes RB jedoch vor der Akquisition des Ultraschall-Volumendatensatzes 3D-US, da so bereits parallel ein folgender vierter Schritt IV ausgeführt werden kann.

Im vierten Schritt IV beginnt das eigentliche Ausführungsbeispiel des erfindungsgemäßen Verfahrens zur Ermittlung eines darzustellenden interessierenden Bereichs dessen Schritte im Detail auch noch anhand von FIG 4 und 5 erläutert werden. Der vierte Schritt IV beschreibt eine Ermittlung einer Projektionsposition PR1, PR2 Die Ermittlung kann beispielsweise durch eine manuelle Eingabe eines Benutzers BE erfolgen. Zusätzlich oder alternativ kann zu Ermittlung auch ein CAD-Algorithmus ausgeführt werden, der unter Verwendung bekannter Methoden automatisch den interessierenden Bereich und gegebenenfalls auch die Projektionsposition PR1, PR2 detektiert. Die Projektionsposition PR1, PR2 entspricht dabei einem Punkt der Detektorfläche D.

In einem fünften Schritt V wird ein Teildatensatz CBV1, CBV1', CBV1" CBV2 aus dem Ultraschall-Volumendatensatz 3D-US extrahiert. Dies erfolgt auf Basis der Projektionsposition PR1, PR2 und einer von geometrischen Informationen GI, die das XUS-Gerät 2 betreffen, wie im Folgenden mit zwei Beispielen anhand von FIG 4 und FIG 5 erläutert wird.

FIG 4 zeigt beispielhaft eine schematische perspektivische Darstellung zur Veranschaulichung der Extraktion zweier Teildatensätze CBV1, CBV2. Die Röntgenquelle Q ist in einem senkrechten Abstand bzw. einer Höhe h über der Detektorfläche D angeordnet. Der Detektor 5 ist eine Vielzahl von Pixeln auf die nach Art einer Matrix in der Detektionsfläche D angeordnet sind. Die Pixel sind dabei senkrecht zueinander stehenden Zeilen und Spalten gegliedert, die sich in den Hauptrichtungen des Detektors 5 erstrecken (hier zur besseren Darstellung nicht gezeigt). Auf der Detektorfläche D ist zu Veranschaulichung ein Umriss der Brust B dargestellt. Zudem ist zur leichteren Orientierung ein kartesisches, rechtshändiges Koordinatensystem mit einer x-Richtung, einer y-Richtung und einer z-Richtung gezeigt. Die z-Richtung entspricht einer Richtung von der Röntgenquelle Q zu einem Projektionspunkt der Röntgenquelle Q auf der Detektorfläche D. Die y-Richtung entspricht einer Hauptrichtung des Detektors 5, die im Wesentlichen von der Patientin weg weist und die x-Richtung, die der anderen Hauptrichtung des Detektors 5 entspricht ergibt sich aus einer Ergänzung der beiden vorherigen Richtungen zu einem rechthändigen kartesischen Koordinatensystem.

Die im vierten Schritt IV ermittelte erste Projektionsposition PR1 entspricht einer Position bzw. einem Pixel der Detektorfläche D. Von der Röntgenquelle Q bis zu der ersten Projektionsposition PR1 erstreckt sich ein erster Projektionsstrahl R1. Eine Länge des ersten Projektionsstrahls R1 entspricht dem Abstand a1 zwischen der Röntgenquellen Q und der Projektionsposition PR1. Die Lage des ersten Projektionsstrahls R1 in Relation zu dem Ultraschall-Volumendatensatz 3D-US kann mithilfe der geometrischen Informationen GI zugeordnet werden. D.h. die Lage des ersten Projektionsstrahls R1 im Verhältnis zu der Detektorfläche D und der Röntgenquelle Q kann ermittelt werden, da auch die relative Lage der Röntgenquelle Q zur Detektorfläche D bekannt ist oder zumindest ermittelt werden kann. Dazu können die Positionen der Röntgenquelle Q und die Detektorfläche D beispielsweise in einem gemeinsamen Koordinatensystem (x, y, z) beschrieben werden.

Aus dem Ultraschall-Volumendatensatz 3D-US wird der zur Projektionsposition PR1 gehörige erste Teildatensatz CBV1 wie folgt ausgewählt. Zunächst wird eine Ebene konstruiert, die durch einen ersten Richtungsvektor U1 und einen zweiten Richtungsvektor V1 aufgespannt wird. Der erste Richtungsvektor U1 erstreckt sich dabei in einer Richtung von dem Projektionspunkt PQ der Röntgenquellen Q zur ersten Projektionsposition PR1. der zweite Richtungsvektor V1 erstreckt sich hier (zur besseren Darstellung und ohne Beschränkung der Allgemeinheit) in einer Richtung entgegen der z-Richtung (einfacher Weise kann sich der zweite Richtung Vektor V1 ebenso auch in z-Richtung erstrecken). Der erste Teildatensatz CBV1 (hier lediglich schematisch und ausschnittweise dargestellt) wird folgend aus einer Schnittmenge zwischen der zuvor beschriebenen Ebene und dem Ultraschall-Volumendatensatz 3D-US gebildet. Dabei umfasst der Teildatensatz aufgrund seiner Konstruktion jedenfalls die Daten die den vom Projektionsstrahl R1 durchdrungenen Bereichen entsprechen. Weiterhin ist es möglich, den ersten Teildatensatz CBV1 zusätzlich zu verkleinern, in dem nur ein definierter und für eine Befundung ausreichender Bereich der Schnittmenge extrahiert wird, der die Projektionsposition, bevorzugt mittig, umfasst.

Für die zweite Projektionsposition PR2 ist zur weiteren Veranschaulichung eine Konstruktion zur Extraktion des zweiten Teildatensatzes CBV2 dargestellt, die anhand der eben zum ersten Teildatensatz CBV1 gemachten Erläuterungen analog nachvollzogen werden kann.

FIG 5 zeigt beispielhaft eine schematische perspektivische Darstellung zur Veranschaulichung der Extraktion zweier Teildatensätze CBV1', CBV1''. FIG 5 ist im Wesentlichen ähnlich zu FIG 4, weswegen im Folgenden lediglich die unterschiedliche Extraktion der Teildatensätze CBV1', CBVl"' näher erläutert wird.

Die Lage des Projektionsstrahls R1 in Relation zum Ultraschall-Volumendatensatz ist analog zu FIG 4 bekannt. Allerdings wird nun zur Auswahl des dritten Teildatensatzes CBV1' keine Ebene konstruiert, sondern ein Kegelstumpf. Der Kegelstumpf repräsentiert dabei die kegelförmige Strahlcharakteristik bzw. Strahlengeometrie der Röntgenquelle Q. Er weist eine gedachte Spitze, eine Mittelachse und einen Öffnungswinkel auf. Die gedachte Spitze ist an derselben Position wie die Röntgenquelle Q angeordnet. Die Mittelachse erstreckt sich in einer Richtung des Projektionsstrahls R1 durch Mittelpunkte von zu ihr senkrechten Querschnittsflächen des Kegelstumpfes. Der Öffnungswinkel bezeichnet einen Öffnungswinkel des Kegels, zu dem die Mittelachse eine Winkelhalbierende ist. Er beträgt hier beispielsweise 10°. Die Projektionsposition P1 ist mittig in dem Kegelstumpf angeordnet. D.h. der Kegelstumpf erstreckt sich in Richtung des Projektionsstrahls und entgegengesetzt zu beiden Seiten der Projektionsposition PR1 in einem definierten Bereich. Der dritte Teildatensatz CBV1' wird aus einer Schnittmenge des derart konstruierten Kegelstumpfes und dem Ultraschall-Volumendatensatz 3D-US gebildet.

Zur einfacheren Darstellung des dritten Teildatensatzes CBV1', können zusätzliche Daten aus benachbarten Bereichen des Ultraschall-Volumendatensatzes 3D-US hinzugenommen werden. Dementsprechend wird ein vierter Teildatensatz CBV1" extrahiert, der, bevorzugt als quaderförmige Bounding-Box (Begrenzungsquader), den dritten Teildatensatz CBV1' in einem größer als der Kegelstumpf definierten Bereich des Ultraschall-Volumendatensatzes 3D-US umfasst. Der vierte Teildatensatz CBVL" ist dabei bevorzugt so angeordnet, dass seine Haupterstreckungsrichtungen den x-y-z-Richtungen des zuvor beschriebenen kartesischen Koordinatensystems entsprechen.

Die zuvor beschriebenen definierten Bereiche für die Teildatensätze CBV1', CBVL" sind bevorzugt so festgelegt, dass sie einem Volumen von höchstens 3 cm³, bevorzugt höchstens 2 cm³, besonders bevorzugt höchstens 1 cm³ entsprechen.

Die Teildatensätze CBV1, CBV1', CBV1", CBV2 werden auch als Cone-Beam-Views bezeichnet und können separat gespeichert werden. Sie stellen eine schneller zu befundende Teilmenge des Ultraschall-Volumendatensatzes 3D-US dar.

Zu FIG 4 und 5 sei angemerkt sie keinesfalls maßstäblich dargestellt sind. So ist die Höhe h der Röntgenquellen Q über der Detektorfläche D üblicherweise wesentlich größer als hier dargestellt. Auch Anordnung wurde zur besseren Darstellung vereinfacht. Anders als dargestellt ist die Röntgenquelle Q bevorzugt möglichst zentral über der Detektorfläche D angeordnet.

Die derart extrahierten Teildatensätze CBV1, CBV1', CBV1", CBV2 werden in den folgenden nun wieder anhand von FIG 3 beschriebenen Schritten VI und VII gemäß einem Ausführungsbeispiel eines erfindungsgemäßen Verfahrens zur Darstellung eines interessierenden Bereichs dargestellt. Dazu wird zunächst in einem sechsten Schritt VI eine Anzahl von Ansichten bereitgestellt. Das Bereitstellen kann beispielsweise erfolgen, indem die Ansichten direkt aus der Bildverarbeitungseinheit empfangen oder aus einem Speicher abgerufen werden. Bei den Teildatensätzen CBV1, CBV2 kann zweidimensionalen Bild zum Beispiel noch ein anzuzeigender Bereich ausgewählt werden. Bei den Teildatensätzen CBV1', CBVL" beispielsweise noch eine Blickrichtung auf den dreidimensionalen Volumendatensatz festgelegt und entsprechende Ansichten generiert werden.

Liegen wie beispielsweise bei den Teildatensätzen CBV1', CBVL" mehrere Ansichten 30 vor, können in einem Schritt VII eine oder mehrere (bei gleichzeitiger Darstellung) der Ansichten 30 für die Darstellung ausgewählt werden. So kann als erste an sich beispielsweise eine Ansicht 30 gewählt werden, die die Projektionsposition umfasst. Weitere Ansichten können beispielsweise aufeinanderfolgende Sequenz z. B. tiefenabhängig geordneter Bilder, insbesondere als Film oder gesteuert durch Benutzereingaben wie beispielsweise Scrollen oder dergleichen dargestellt werden.

Die einzige Darstellung erfolgt in einem achten Schritt VIII, indem die Anzahl der darzustellenden Ansichten 30 an einer Anzeigeeinheit, wie beispielsweise den Bildschirm 15, übermittelt werden.

Eine solche Ansicht 30 ist beispielhaft in FIG 6 dargestellt. In einem interessierenden Bereich des Untersuchungsobjekts B wurde im Röntgenprojektionsbild RB die Projektionsposition PR1 ermittelt, die auch in der Ansicht 30 eines erfindungsgemäß ermittelten Teildatensatzes mittels eines Fadenkreuzes 33 visualisiert ist. Ebenfalls ist die Lage des Projektionsstrahls R1 relativ zu der Ansicht 30 markiert. Der Teildatensatz umfasst dabei zumindest die Daten des Ultraschall-Volumendatensatzes 3D-UV, die aus einem Korridor 31 des Untersuchungsobjekts B stammen der Korridor 31 erstreckt in der Ebene der Ansicht 30 (die im Wesentlichen senkrecht zur Hauptausbreitungsrichtung der Ultraschallwellen angeordnet ist) und parallel zum Projektionsstrahl R1. Der Korridor weist eine Korridorbreite 32 von 2 cm auf.

Die vorliegende Erfindung ermöglicht somit eine einfache schnelle Befundung, da nicht der gesamte Ultraschall-Volumendatensatz betrachtet werden muss, sondern eine Vorauswahl des interessierenden Bereichs in dem Röntgenprojektionsbild erfolgt. Da nur ein Röntgenprojektionsbild als Übersichtsaufnahme akquiriert werden muss, ist die Strahlenbelastung für den Patienten vorteilhaft gering.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. So wurde zuvor beispielhaft lediglich ein Mammographiesystem beschrieben, die Erfindung kann sich jedoch grundsätzlich auf jedes Röntgensystem mit kombiniertem Röntgen-3D-Ultraschall-Gerät beziehen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einrichtung", "Einheit", "Anordnung" und "System" nicht aus, dass die betreffende Komponente aus mehreren zusammenwirkenden Teilkomponenten besteht, die gegebenenfalls auch räumlich verteilt sein können.

## Patentansprüche

1. Von einem Computer (20) ausgeführtes Verfahren zur Ermittlung eines darzustellenden interessierenden Bereichs in einem Ultraschall-Volumendatensatz (3D-US) vom Inneren eines Untersuchungsobjekts (B)umfassend zumindest folgende Schritte:
- automatisches ermitteln einer Projektionsposition (PR1, PR2) in einem zweidimensionalen Röntgenprojektionsbild (RB) des Untersuchungsobjekts (B), wobei ein der Projektionsposition (PR1, PR2) zugeordneter Projektionsstrahl (R1, R2) durch den interessierenden Bereich verläuft und
- Extraktion eines vom Ultraschall-Volumendatensatz (3D-US) umfassten Teildatensatzes (CBV1, CBV1', CBV1", CBV2) anhand der Projektionsposition (PR1, PR2) und auf Basis von geometrischen Informationen (GI) betreffend das Röntgen-3D-Ultraschall-Gerät (2), wobei die Auswahl der Daten gemäß einer Korrelation zwischen der Projektionsposition und zumindest einem Teil der Daten des Ultraschall-Volumendatensatzes erfolgt, welche der Projektionsposition entsprechen.

2. Verfahren nach Anspruch 1, wobei der Teildatensatz (CBV1, CBV1', CBV1", CBV2) zumindest Teildaten umfasst, die Bereichen des Untersuchungsobjekts (B) zugeordnet werden, die vom Projektionsstrahl (R1, R2) durchdrungen wurden.

3. Verfahren nach Anspruch 2, wobei der Teildatensatz (CBV1, CBV2) als eine Fläche ausgebildet ist.

4. Verfahren nach Anspruch 1 oder 2, wobei der Teildatensatz (CBV1`; CBV1'') Daten aus einem definierten Volumenbereich umfasst.

5. Verfahren nach Anspruch 4, wobei der Volumenbereich einen Korridor (31) durch das Untersuchungsobjekt (B) umfasst, der sich in einer ersten Richtung des Projektionsstrahls (R1, R2) erstreckt und eine Korridorbreite (32) von höchstens 2 cm, bevorzugt höchstens 1 cm, aufweist.

6. Verfahren nach einem der Ansprüche 4 oder 5, wobei der Teildatensatz Teildaten umfasst, die einem Volumen von höchstens 3 cm³, bevorzugt höchstens 2 cm³, besonders bevorzugt höchstens 1 cm³ entsprechen.

7. Verfahren nach einem der vorstehenden Ansprüche, wobei der Teildatensatz mittels einer automatischen Detektion und/oder einer automatischen Klassifizierung des interessierenden Bereichs analysiert wird.

8. Verfahren nach einem der vorstehenden Ansprüche, wobei der Ultraschall-Volumendatensatz (3D-US) und das zweidimensionalen Röntgenprojektionsbild (RB) während eines Kompressionsschritts des Untersuchungsobjekts (B) akquiriert wurden.

9. Verfahren nach einem der vorstehenden Ansprüche, wobei die geometrischen Informationen (GI) zumindest eine der folgenden Informationen umfassen: Strahlgeometrie, Abstand (h) zwischen Röntgenquelle (Q) und Detektor (D), Dicke des Untersuchungsobjekts (B), Abstand (a1, a2) zwischen Röntgenquelle (Q) und Projektionsposition (PR1, PR2).

10. Verfahren zur Darstellung eines interessierenden Bereich, wobei
- eine Anzahl von Ansichten (30) aus einem Teildatensatz (CBV1, CBV1', CBV1", CBV2), der gemäß eines Verfahrens nach einem der Ansprüche 1 bis 9 ermittelt wurde, bereitgestellt wird und
- zumindest eine Ansicht (30) dargestellt wird.

11. Bildverarbeitungseinrichtung (10) zur Ermittlung eines darzustellenden interessierenden Bereichs in einem Ultraschall-Volumendatensatz (3D-US) vom Inneren eines Untersuchungsobjekts (B), der mittels eines Röntgen-3D-UltraschallGeräts akquiriert wurde, umfassend
- eine Ermittlungseinheit, die ausgebildet ist, eine Projektionsposition (PR1, PR2) in einem zweidimensionalen Röntgenprojektionsbild (RB) des Untersuchungsobjekts (B) automatisch zu ermitteln, wobei ein der Projektionsposition (PR1, PR2) zugeordneter Projektionsstrahl (R1, R2) durch den interessierenden Bereich (ROI) verläuft und
- eine Extraktionseinheit, die ausgebildet ist, einen vom Ultraschall-Volumendatensatz (3D-US) umfassten Teildatensatzes (CBV1, CBV1', CBV1", CBV2) anhand der Projektionsposition (PR1, PR2) und auf Basis von geometrischen Informationen (GI) betreffend das Mammographie-3D-Ultraschall-Gerät (2) zu extrahieren, wobei die Auswahl der Daten gemäß einer Korrelation zwischen der Projektionsposition und zumindest einem Teil der Daten des Ultraschall-Volumendatensatzes erfolgt, welche der Projektionsposition entsprechen.

12. Röntgensystem (1) mit einer Bildverarbeitungseinrichtung (10) nach Anspruch 11 und einem Röntgen-3D-Ultraschall-Gerät (2) umfassend
- eine Quelle-Detektor-Anordnung (3), die zur Aufnahme eines Röntgenprojektionsbilds (RB) eines Untersuchungsobjekts (B) ausgebildet ist und
- eine Ultraschallanordnung (6), die zur Aufnahme eines Ultraschall-Volumendatensatzes (3D-US) ausgebildet ist.

13. Computerprogrammprodukt mit einem Computerprogramm, welches direkt in eine Speichereinrichtung einer Bildverarbeitungseinrichtung (10) ladbar ist, mit Programmabschnitten, um alle Schritte eines Verfahrens nach einem der Ansprüche 1 bis 10 auszuführen, wenn das Computerprogramm in der Bildverarbeitungseinrichtung (10) ausgeführt wird.

14. Computerlesbares Medium, auf welchem von einer Rechnereinheit einlesbare und ausführbare Programmabschnitte gespeichert sind, um alle Schritte eines Verfahrens nach einem der Ansprüche 1 bis 10 auszuführen, wenn die Programmabschnitte von der Rechnereinheit ausgeführt werden.

## Claims

1. Method carried out by a computer (20) for determining a region of interest to be rendered in an ultrasound volume data set (3D-US) of the interior of an object under examination (B), comprising at least the following steps:
- automatically determining a projection position (PR1, PR2) in a two dimensional X-ray projection image (RB) of the object under examination (B), wherein a projection ray (R1, R2) assigned to the projection position (PR1, PR2) passes through the region of interest and
- extracting a partial data set (CBV1, CBV1', CBV1 ", CBV2) encompassed by the ultrasound volume data set (3D-US) using the projection position (PR1, PR2) and on the basis of geometrical information (GI) relating to the X-ray 3D ultrasound unit (2), wherein the selection of the data takes place according to a correlation between the projection position and at least some of the data of the ultrasound volume data set corresponding to the projection position.

2. Method according to claim 1, wherein the partial data set (CBV1, CBV1', CBV1 ", CBV2) at least comprises partial data which is assigned to regions of the object under examination (B) that have been penetrated by the projection ray (R1, R2).

3. Method according to claim 2, wherein the partial data set (CBV1, CBV2) is implemented as a surface.

4. Method according to claim 1 or 2, wherein the partial data set (CBV1`; CBV1") comprises data from a defined volume range.

5. Method according to claim 4, wherein the volume range encompasses a corridor (31) through the object under examination (B) that extends in a first direction of the projection ray (R1, R2) and has a corridor width (32) of no more than 2 cm, preferably of no more than 1 cm.

6. Method according to one of claims 4 or 5, wherein the partial data set comprises partial data corresponding to a volume of no more than 3 cm³, preferably of no more than 2 cm³, with particular preference of no more than 1 cm³.

7. Method according to one of the preceding claims, wherein the partial data set is analysed by means of automatic detection and/or automatic classification of the region of interest.

8. Method according to one of the preceding claims, wherein the ultrasound volume data set (3D-US) and the two dimensional X-ray projection image (RB) have been acquired during a compression step of the object under examination (B).

9. Method according to one of the preceding claims, wherein the geometrical information (GI) comprises at least one of the following items of information: beam geometry, distance (h) between X-ray source (Q) and detector (D), thickness of the object under examination (B), distance (a1, a2) between X-ray source (Q) and projection position (PR1, PR2).

10. Method for rendering a region of interest, wherein
- a number of views (30) from a partial data set (CBV1, CBV1', CBV1", CBV2) that has been determined according to a method according to one of claims 1 to 9 are provided and
- at least one view (30) is rendered.

11. Image processing device (10) for determining a region of interest to be rendered in an ultrasound volume data set (3D-US) of the inside of an object under examination (B), said data set having been acquired by means of an X-ray 3D ultrasound unit, comprising
- a determination unit which is designed to automatically determine a projection position (PR1, PR2) in a two dimensional X-ray projection image (RB) of the object under examination (B), wherein a projection ray (R1, R2) assigned to the projection position (PR1, PR2) passes through the region of interest (ROI) and
- an extraction unit which is designed to extract a partial data set (CBV1, CBV1', CBV1", CBV2) from the ultrasound volume data set (3D-US) using the projection position (PR1, PR2) and on the basis of geometrical information (GI) relating to the mammography 3D ultrasound unit (2), wherein the selection of the data takes place according to a correlation between the projection position and at least some of the data of the ultrasound volume data set corresponding to the projection position.

12. X-ray system (1) having an image processing device (10) according to claim 11 and an X-ray 3D ultrasound unit (2) comprising
- a source-detector arrangement (3) which is designed to obtain an X-ray projection image (RB) of an object under examination (B) and
- an ultrasound arrangement (6) which is designed to acquire an ultrasound volume data set (3D-US).

13. Computer program product comprising a computer program which can be loaded directly into a storage device of an image processing device (10), having program sections for carrying out all the steps of a method according to one of claims 1 to 10 when the computer program is executed in the image processing device (10).

14. Computer-readable medium on which program sections readable and executable by the processing unit are stored in order to carry out all the steps of a method according to one of claims 1 to 10 when the program sections are executed by the processing unit.

## Revendications

1. Procédé exécuté par ordinateur (20) de détermination d'une partie intéressante à représenter dans un ensemble (3D-US) de données en volume d'ultrasons de l'intérieur d'un objet (B) en examen, comprenant au moins les stades suivants :
- détermination automatique d'une position (PR1, PR2) de projection dans une image (RB) de projection aux rayons X en deux dimensions de l'objet (B) en examen, dans lequel un rayon (R1, R2) de projection associé à la position (PR1, PR2) de projection traverse la partie intéressante et
- extraction d'un ensemble (CBV1, CBV1', CBV1", CBV2) de données partielles compris par l'ensemble (3D-US) de données en volume d'ultrasons, à l'aide de la position (PR1, PR2) de projection et sur la base d'informations (GI) géométriques concernant l'appareil (2) d'ultrasons en 3D à rayons X, dans lequel le choix des données s'effectue conformément à une corrélation entre la position de projection et au moins une partie des données de l'ensemble de données en volume d'ultrasons, qui correspondent à la position de projection.

2. Procédé suivant la revendication 1, dans lequel l'ensemble (CBV1, CBV1', CBV1", CBV2) de données partielles comprend au moins des données partielles, qui sont associées à des parties de l'objet (B) en examen, qui ont été traversées par le rayon (R1, R2) de projection.

3. Procédé suivant la revendication 2, dans lequel l'ensemble (CBV1, CBV2) de données partielles est constitué sous la forme d'une surface.

4. Procédé suivant la revendication 1 ou 2, dans lequel l'ensemble (CBV1' ; CBV1'') de données partielles comprend des données à partir d'une partie en volume déterminée.

5. Procédé suivant la revendication 4, dans lequel la partie en volume comprend un corridor (31) dans l'objet (B) en examen, qui s'étend dans une première direction du rayon (R1, R2) de projection et a une largeur (32) de corridor de 2 cm au plus, de préférence de 1 cm au plus.

6. Procédé suivant l'une des revendications 4 ou 5, dans lequel l'ensemble de données partielles comprend des données partielles, qui correspondent à un volume de 3 cm³ au plus, de préférence de 2 cm³ au plus, d'une manière particulièrement préférée de 1 cm³ au plus.

7. Procédé suivant l'une des revendications précédentes, dans lequel on analyse l'ensemble de données partielles, au moyen d'une détection automatique et/ou d'une classification automatique de la partie intéressante.

8. Procédé suivant l'une des revendications précédentes, dans lequel on a acquis l'ensemble (3D-US) de données en volume d'ultrasons et l'image (RB) de projection aux rayons X en deux dimensions pendant un stade de compression de l'objet (B) en examen.

9. Procédé suivant l'une des revendications précédentes, dans lequel les informations (GI) géométriques comprennent au moins l'une des informations suivantes : géométrie du rayon, distance (h) entre source (Q) de rayons X et détecteur (D), épaisseur de l'objet (B) en examen, distance (a1, a2) entre source (Q) de rayons X et position (PR1, PR2) de projection.

10. Procédé de représentation d'une partie intéressante, dans lequel
- on se procure un nombre de vues (30) à partir d'un ensemble (CBV1, CBV1', CBV1", CBV2) de données partielles, qui a été déterminé selon un procédé suivant l'une des revendications 1 à 9, et
- on représente au moins une vue (30).

11. Dispositif (10) de traitement d'image pour la détermination d'une partie intéressante à représenter dans un ensemble (3D-US) de données en volume d'ultrasons de l'intérieur d'un objet (B) en examen, qui a été acquis au moyen d'un appareil d'ultrasons en 3D à rayons X, comprenant
- une unité de détermination, qui est constituée pour déterminer automatiquement une position (PR1, PR2) de projection dans une image (RB) de projection aux rayons X en deux dimensions de l'objet (B) en examen, dans lequel un rayon (R1, R2) de projection associé à la position (PR1, PR2) de projection traverse la partie (ROI) intéressante, et
- une unité d'extraction, qui est constituée pour extraire un ensemble (CBV1, CBV1', CBV1", CBV2) de données partielles compris par l'ensemble (3D-US) de données en volume d'ultrasons, à l'aide de la position (PR1, PR2) de projection et sur la base d'informations (GI) géométriques concernant l'appareil (2) d'ultrasons en 3D de mammographie, dans lequel le choix des données s'effectue conformément à une corrélation entre la position de projection et au moins une partie des données de l'ensemble de données en volume d'ultrasons, qui correspondent à la position de projection.

12. Système (1) à rayons X, comprenant un dispositif (10) de traitement d'image suivant la revendication 11
et un appareil (2) d'ultrasons en 3D a rayons x, comprenant
- un agencement (3) source-détecteur, qui est constitué pour l'enregistrement d'une image (RB) de projection à rayons X d'un objet (B) en examen et
- un agencement (6) d'ultrasons, qui est constitué pour l'enregistrement d'un ensemble (3D-US) de données en volume d'ultrasons.

13. Produit de programme d'ordinateur, comprenant un programme d'ordinateur, qui peut être chargé directement dans un dispositif de mémoire d'un dispositif (10) de traitement d'image, comprenant des parties de programme pour exécuter tous les stades d'un procédé suivant l'une des revendications 1 à 10, lorsque le programme d'ordinateur est exécuté dans le dispositif (10) de traitement d'image.

14. Support déchiffrable par ordinateur, sur lequel sont mises en mémoire des parties de programme pouvant être lues et exécutées par une unité informatique, afin d'exécuter tous les stades d'un procédé suivant l'une des revendications 1 à 10, lorsque les parties de programme sont exécutées par l'unité informatique.
